# EUROPÄISCHE PATENTSCHRIFT

(11) **EP 0 979 083 B1**
(45) Veröffentlichungstag und Bekanntmachung des Hinweises auf die Patenterteilung: **13.08.2003**
(21) Anmeldenummer: 98933532.8
(22) Anmeldetag: 14.05.1998
(51) Int. Cl.: A61K 31/535, A61P 25/22

(54) **VERWENDUNG VON 1-AR(ALK)YL-IMIDAZOLIN-2-ONE ZUR BEHANDLUNG VON ANGST- UND SPANNUNGSZUSTÄNDEN**
USE OF 1-AR(ALK)YL-IMIDAZOLIN-2-ONE FOR TREATING ANXIETY AND STRESS CONDITIONS
UTILISATION DE 1-AR(ALK)YL-IMIDAZOLIN-2-ONE POUR LE TRAITEMENT DE L'ANGOISSE ET DU STRESS

(30) Priorität: 23.05.1997 DE 19721580
(43) Veröffentlichungstag der Anmeldung: 16.02.2000
(73) Patentinhaber: Arzneimittelwerk Dresden GmbH, 01445 Radebeul (DE)
(72) Erfinder: ROSTOCK, Angelika, D-01445 Radebeul (DE); DOST, Rita, D-01217 Dresden (DE); TOBER, Christine, D-01689 Weinböhla (DE); BARTSCH, Reni, D-01458 Ottendorf-Okrilla (DE); UNVERFERTH, Klaus, D-01309 Dresden (DE); RUNDFELDT, Chris, D-01640 Coswig (DE)
(74) Vertreter: Böhm, Brigitte, Dipl.-Chem. Dr.
(86) Internationale Anmeldenummer: DE9801343
(87) Internationale Veröffentlichungsnummer: WO98052548

(56) Entgegenhaltungen:
- DE-A- 19 532 668
- US-A- 4 044 021
- SIGEL, ERWIN ET AL: "The antiepileptic drug AWD 131-138 stimulates different recombinant isoforms of the GABAA receptor through the benzodiazepine binding site" NEUROSCI. LETT. (1998), 245(2), 85-88 CODEN: NELED5;ISSN: 0304-3940, XP002083597
- ROSTOCK, A. ET AL: "AWD 131-138 as anxiolytic anticonvulsant" DRUGS FUTURE (1998), 23(3), 253-255 CODEN: DRFUD4;ISSN: 0377-8282, XP002083598

## Beschreibung

Die Erfindung betrifft die Verwendung von 1-Ar(alk)yl-imidazolin-2-onen der allgemeinen Formel I oder deren pharmazeutisch verwendbaren Salze zur Herstellung eines Arzneimittels zur Behandlung von Angst- und Spannungszuständen.

Die Verbindungen der allgemeinen Formel I 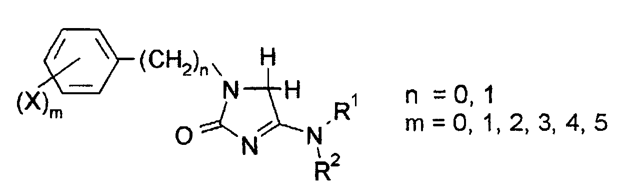 worin
X = Wasserstoff, C₁-C₄-Alkyl, C₁-C₄-Alkoxy, Trifluormethyl, Halogen ,
R¹ bzw. R² = C₁-C₄-Alkyl, Cycloalkyl, Heteroalkyl
oder
R¹ und R² zusammen eine Alkylengruppe mit 2-6 Kohlenstoffatomen in dem eine -CH₂-Gruppe durch Sauerstoff, Stickstoff oder Schwefel ersetzt sein kann, bedeuten und die Anzahl der CH₂-Gruppen entweder 0 (1-Aryl-imidazolin-2-one) oder 1 (1-Aralkyl-imidazolin-2-one) beträgt sind in der deutschen Patentanmeldung 195 32 668.7 beschrieben.

Pharmakologische Ergebnisse zeigen, daß die Verbindungen der allgemeinen Formel 1 zur Behandlung von verschiedenen Arten epileptischer Erkrankungen geeignet sind.

Angst- und Spannungszustände unterschiedlicher Ursache und Ausprägung können auch heute nicht in allen Fällen zufriedenstellend behandelt werden. Seit etwa 1960 werden zur Behandlung von Angst- und Spannungszuständen vorrangig Benzodiazepin-Derivate eingesetzt. Substanzen mit einem solchen Profil wirken im allgemeinen beruhigend und affektdämpfend. Kurzfristig helfen diese Medikamente gut, aber bereits in therapeutischen Dosierungen treten Nebenwirkungen wie Sedation, Schläfrigkeit sowie verringerte Reaktionsfähigkeit auf.

Durch die Sedierung kann es zu einer negativen Beeinflussung mentaler Prozesse kommen. Zum Teil sind Ataxie und Koordinationstörungen zu beobachten, welche das Leistungsvermögen beeinflussen.
Bei Dauergebrauch führen diese Benzodiazepin-Verbindungen zu Gewöhnungseffekten, der sogenannten Toleranz. Die Wirksamkeit des Präparates wird geringer und die Dosis muß erhöht werden. Es kann sich eine psychische, darüber hinaus auch eine physische Abhängigkeit entwickeln.
Bei Absetzversuchen treten deshalb komplizierte Entzugsphänomene auf.

Als wichtigste Vertreter der am Markt eingeführten Anxiolytika sind die Wirkstoffe Diazepam, Clonazepam und Medazepam zu nennen.
Um eine anxiolytische Wirkung von Diazepam zu erzielen, sind Plasmakonzentrationen von 300 bis 400 mg/ml notwendig. Bei gleichen Konzentrationen treten aber auch die erwähnten Nebenwirkungen, wie Sedation und psychomotorische Störungen auf, die sich in Tagessedation, Schläfrigkeit sowie eingeschränkte Aufmerksamkeit und Reaktionsfähigkeit äußern. Auf Grund der hohen Halbwertszeit von Diazepam und Clonazepam, treten starke "Hang-over"-Effekte auf, die ebenfalls mit Schläfrigkeit, Verschlechterung von intellektuellen und motorischen Leistungen sowie verlängerter Reaktionszeit verbunden sind. Die anxiolytische Wirkung von Clonazepam wird von der sedierenden beziehungsweise hypnotischen Wirkung verdeckt. Auch hohe Dosen von Medazepam sind mit hypnotischen, muskelrelaxierenden Erscheinungen verbunden.

Alle drei Medikamente verstärken die Wirkung zahlreicher zentral wirkender Pharmaka und von Alkohol. Dabei können Effekte auftreten, die nach Verabreichung der Einzelsubstanzen kaum zu bemerken sind.

Es ist bisher nicht gelungen, bei länger andauernden Angstzuständen einen befriedigenden therapeutischen Standard zu erreichen. Auch ist eine therapieüberdauemde Wirkung angstlösender Medikamente gegenwärtig nicht ausreichend gewährleistet.

Aufgabe der vorliegenden Erfindung ist es deshalb, Arzneimittel zur Behandlung von unterschiedlichen Angst- und Spannungszustände bereitzustellen, welche eine große therapeutische Breite aufweisen.

Überraschenderweise wurde gefunden, daß Verbindungen der allgemeinen Formel I bedeutende anxiolytische Wirkungen im Tierexperiment ohne sedierende Effekte aufweisen.

### Pharmakologische Untersuchungen

Ziel der Untersuchungen mit Verbindungen der allgemeinen Formel I ist es, in Modellen zur Untersuchung der Wirkung gegen Angstzustände die mögliche Beeinflussung abzuschätzen. Dazu wurden die Tiere unterschiedlichen Konfliktsituationen ausgesetzt und eine Beeinflussung beispielsweise durch die Verbindung 1-(4-Chlorphenyl)-4-morpholino-imidazolin-2-on (Beispiel 1) gemessen.

### Untersuchung der Angsthemmung im Vogel-Konflikt-Test

In diesem Modell wird Ratten über eine gewisse Zeit der ständige Zugang zum Trinkwasser vorenthalten. Nach dieser Periode ist der Zugang zum Trinkwasser freigegeben, wird jedoch mit einem schwachen elektrischen Reiz gekoppelt.
Für die Tiere entsteht der Konflikt, den elektrischen Reiz in Kauf zu nehmen oder auf das Trinken zu verzichten.

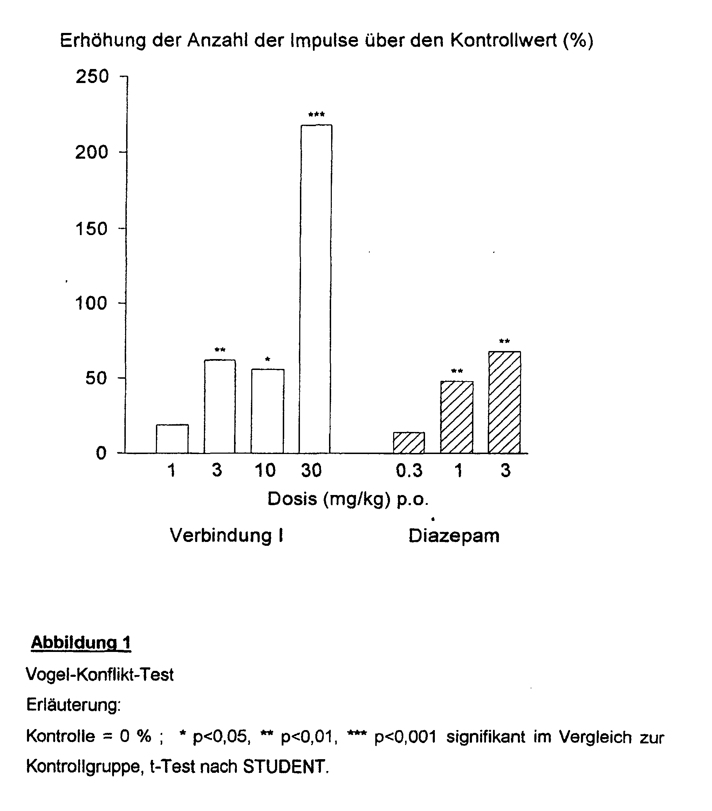

Die Reaktionen auf eine derartige Konfliktsituation ähneln den Folgeerscheinungen der Angst beim Menschen. Es entstehen Vermeidungsreaktionen, die durch anxiolytisch wirkende Substanzen unterdrückt werden können. Als Maß der anxiolytischen Wirkung wird die Zahl der tolerierten Stromstöße der mit Substanz behandelten Tiere im Vergleich zur Vehikel-behandelten Kontrollgruppe bewertet. Die erhaltenen Versuchsergebnisse gemäß Tabelle 1 sind in der Abbildung 1 graphisch dargestellt.

**Tabelle 1**

| **Anxiolytische Wirkung von Substanzen im Vogel-Konflikt-Test/Ratte** | | | |
|---|---|---|---|
| **Substanz** | **mg/kg p.o.** | **Impulse** | **Veränderte Anzahl der Impulse zur Kontrolle in %** |
| Kontrolle | - | 51,4 ± 7,73 | |
| | | | |
| **Beispiel 1** | 1 | 61,3 ± 8,72 | 19,3 |
| | 3 | 83,2 ± 7,19 ** | 61,9 |
| | 10 | 80,1 ± 9,23** | 55,8 |
| | | | |
| | - | 39,6± 7,20 | |
| Kontrolle | 30 | 126,0 ± 15,7 ** | 218,0 |
| Kontrolle | - | 62,3 ± 7,07 | |
| | | | |
| **Diazepam** | 0,1 | 58,4 ± 6,47 | -6,3 |
| | 0,3 | 70,9 ± 6,85 | 13,8 |
| | 1,0 | 92,1 ± 3,22 ** | 47,8 |
| | 3,0 | 104,4 ± 11,9 ** | 67,6 |
| Kontrolle | - | 63,9 ± 6,63 | |
| | | | |
| **Clonazepam** | 0,1 | 77,7 ± 8,54 | 21,6 |
| | 0,3 | 81,8 ± 7,81 | 28,0 |
| | 1,0 | 110,3 ± 13,5 * | 72,6 |
| Kontrolle | - | 54,6 ± 7,89 | |
| | | | |
| **Medazepam** | 0,3 | 54,8 ± 8,85 | 0,4 |
| | 1,0 | 71,3 ± 10,0 | 30,6 |
| | 3,0 | 42,7 ± 4,54 | -21,8 |
| x ± SEM; | | | |

| | | | |
|---|---|---|---|
| * p < 0,05, | | | |
| ** p < 0,01 | | | |

Für die Verbindung gemäß Beispiel 1 wurde bereits ab 3 mg/kg p.o. eine anxiolytische Wirkung nachgewiesen, die bei Aufdosierung auf 10 mg/kg p.o. nicht erhöht wird.
Nach Dosissteigerung auf 30 mg/kg p.o. konnte eine Wirkungsverstärkung gemessen werden. Äquieffektive Dosen von Diazepam und Clonazepam sind 1 bis 3 mg/kg p.o. und 1 mg/kg p.o.
Für Medazepam konnte im Dosisbereich von 0,3 bis 3 mg/kg p.o. keine Wirkung nachgewiesen werden.

Unbehandelte Tiere trinken wesentlich weniger, das bedeutet, sie sind ängstlicher als Tiere, die mit angsthemmenden Substanzen behandelt sind. Die Verbindung gemäß Beispiel 1 erhöht die Zahl der tolerierten elektrischen Reize signifikant ab der Dosis 3 mg/kg oral. Dieser Effekt belegt die gute anxiolytische Wirkung der Verbindungen der allgemeinen Formel I.

Es ist daher zu erwarten, daß die Verbindung der allgemeinen Formel I. eine Hemmung der Angst besonders in Konfliktsituationen bewirken.

### Untersuchung der Angsthemmung im elevated maze

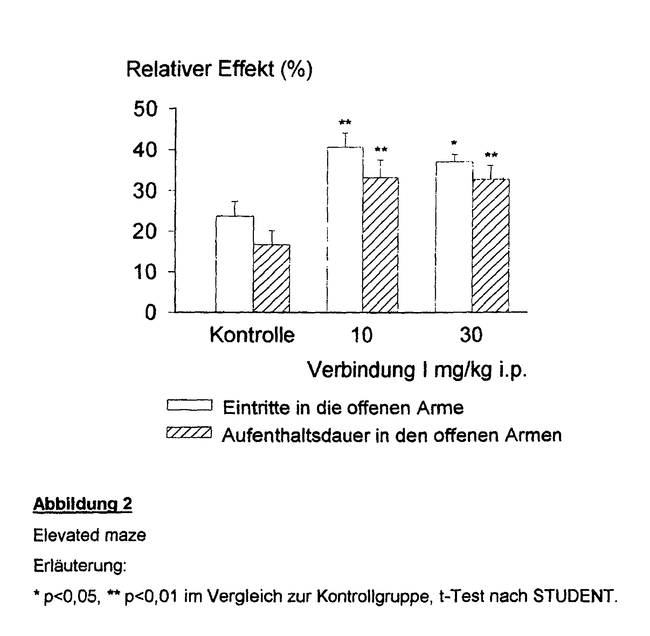

In diesem Modell werden Ratten in ein erhöhtes Gangsystem mit offenen und geschlossenen Armen gesetzt (Pellow, S., Chopin, P., File S. E., Briley, M.: Validation of open: closed arm entries in an elevated plus-maze as a measure of anxiety in rats. J. of Neuroscience Methods 14: 149-167, 1985 ; Hogg, S.: A review of the validity and variability of the elevated plus-maze as an animal model of anxiety. Pharmacology Biochemistry and Behavior: 21-30, 1996).
Unbehandelte Tiere streben vermehrt den geschlossenen Gängen zu. Die Angsthemmung wird an der Anzahl der Eintritte in die offenen Arme und an der Aufenthaltsdauer in den offenen Armen prozentual zu den Gesamteintritten beziehungsweise der gesamt Aufenthaltsdauer gemessen. Behandlungen mit der Verbindungen der allgemeinen Formel 1 erhöhen prozentual die Eintritte und die Aufenthaltsdauer in den offenen Armen, wie es aus der Abbildung 2 ersichtlich ist.

Der Anteil der Eintritte in die offenen Arme und der Aufenthaltszeit in den offenen Armen ist nach intraperitomaler Applikation von 10 und 30 mg/kg der Verbindung gemäß Beispel 1 signifikant erhöht.

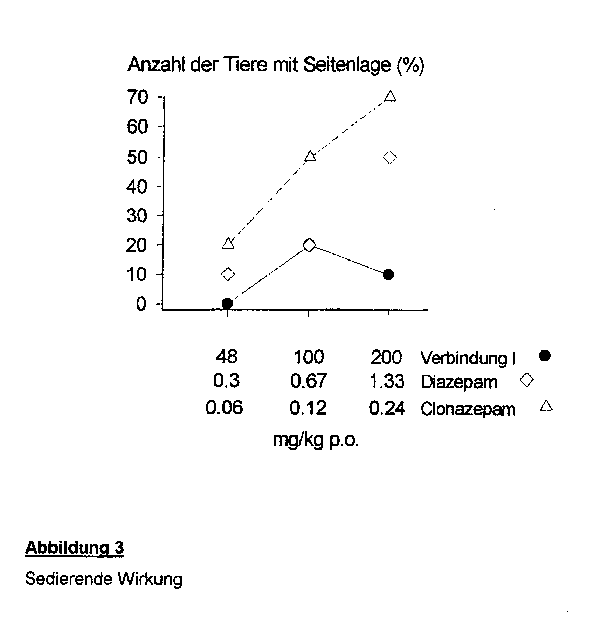

Die Verbindungen der allgemeinen Formel I zeigten im pharmakologischen Experiment eine starke Separierung zwischen der anxiolytischen Wirkung und den sedierenden Effekten.
Aus der Abbildung 3 ist ersichtlich, daß beispielsweise die Verbindung gemäß Beispiel 1 gegenüber der Vergleichssubstanz Diazepam eine wesentlich geringere Sedation aufweist.

Die zentral sedierende Wirkung der Verbindungen der allgemeinen Formel I wurde an Mäusen untersucht. Die Tiere erhielten eine Alkoholmenge appliziert, die keine Seitenlage der Mäuse hervorruft. Es wurde geprüft, inwieweit der hypnotische Effekt von Alkohol verstärkt wird und durch zusätzliche Gabe von Verbindungen der allgemeinen Formel I Seitenlagen bei den Tieren induziert werden kann. Dosierungen beispielsweise der Verbindung gemäß Beispiel 1, die das 15 bis 66 - fache der anxiolytisch wirksamen Dosis betragen, induzieren nur eine geringe und
nicht dosisabhängige Verstärkung der Wirkung von Alkohol. Diazepam und Clonazepam wurden dagegen in anxiolytisch wirksamen Dosisbereich untersucht. Für beide Standardverbindungen konnte eine starke und dosisabhängige Verstärkung der Alkoholwirkung gemessen werden (Abbildung 3).

Die Verbindungen der allgemeinen Formel I weisen auch gegenüber den Vergleichssubstanzen Diazepam und Clonazepam eine sehr geringe Neurotoxizität auf.

Die minimal neurotoxische Dosis der Verbindung gemäß Beispiel 1 wurde im Drehstabtest mit 998 mg/kg p.o. bestimmt.

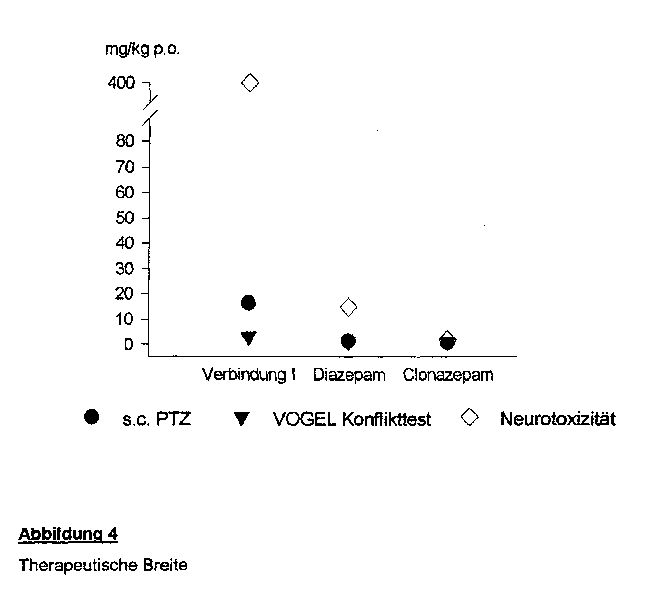

Der therapeutische Index, berechnet als Quotient der minimal neurotoxischen Dosis im Drehstabtest und der anxiolytisch wirksamen Dosis ist mit dem Wert 333 sehr hoch und weist auf eine große therapeutische Breite hin, wie es aus der Abbildung 4 ersichtlich ist.

Die therapeutische Breite einer Substanz als wichtige pharmakologische Kenngröße ist ein Maß für die Sicherheit zwischen therapeutischer und toxischer Wirkung.
Um so überrraschender war deshalb, daß Verbindungen der allgemeinen Formel I gegenüber den am Markt befindlichen anxiolytisch wirksamen Substanzen eine wesentlich höheren therapeutische Breite aufweisen.
Dadurch wird die Behandlung von Patienten mit Angst - und Spannungszuständen , insbesondere auch über einen längeren Zeitraum, deutlich verbessert.

Die Verbindungen der allgemeinen Formel I sowie deren pharmazeutisch verwendbaren Salze können in bekannter Weise in pharmazeutische Formulierungen wie Tabletten, Kapseln, Dragees, Granulate, Emulsionen, Suspensionen oder Lösungen überführt werden.
Zur Herstellung dieser Zubereitungen können die üblichen pharmazeutischen Träger- und Hilfsstoffe verwendet werden.
Dabei sollte die Tagesdosis vorzugsweise 3 - 30 mg/Tag betragen.

## Patentansprüche

1. Verwendung von Verbindungen der allgemeinen Formel I 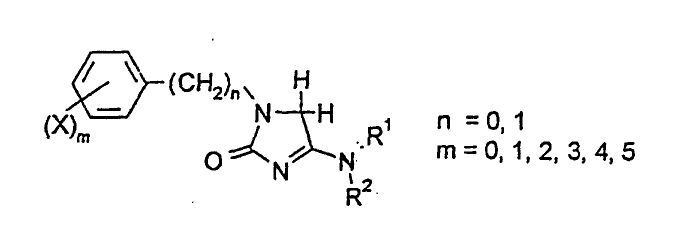 worin
X = Wasserstoff, C₁-C₄-Alkyl, C₁-C₄-Alkoxy, Trifluormethyl, Halogen;
R¹ bzw. R² = C₁-C₄-Alkyl, Cycloalkyl, Heteroalkyl;
oder
R¹ und R² zusammen eine Alkylengruppe mit 2 - 6 Kohlenstoffatomen, in dem eine -CH₂-Gruppe durch Sauerstoff, Stickstoff oder Schwefel ersetzt sein kann, bedeuten und die Anzahl der CH₂-Gruppen entweder 0 (1-Arylimidazolin-2-one) oder 1 (1-Aralkyl-imidazolin-2-one) beträgt; sowie deren pharmazeutisch verwendbaren Salze zur Herstellung eines Arzneimittels zur Behandlung von Angst- und Spannungszuständen.

2. Verwendung der Verbindungen der allgemeinen Formel (I) nach Anspruch 1 zur Herstellung eines Arzneimittels zur Behandlung von konfliktbedingten Angstzuständen.

## Claims

1. Use of compounds of the general formula I 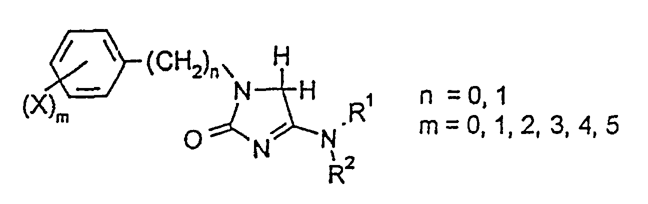 in which
X = hydrogen, C₁-C₄ alkyl, C₁-C₄ alkoxy, trifluoromethyl, halogen,
R¹ or R² = C₁-C₄ alkyl, cycloalkyl, heteroalkyl
or
R¹ and R² together denote an alkylene group with 2-6 carbon atoms in which one CH₂ group can be replaced by oxygen, nitrogen or sulphur and the number of CH₂ groups is either 0 (l-aryl-imidazolin-2-ones) or 1 (1-aralkyl-imidazolin-2-ones) and pharmaceutically acceptable salts thereof to produce a pharmaceutical preparation for treating anxiety and stress states.

2. Use of the compounds of the general formula (I) as claimed in claim 1 to produce a pharmaceutical preparation for treating conflict-induced anxiety states.

## Revendications

1. Utilisation de composés de formule générale I 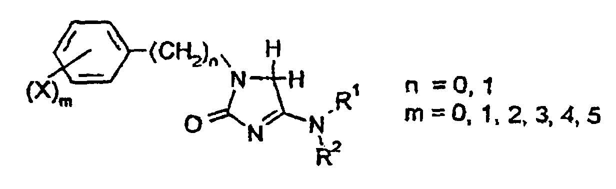 où
X = hydrogène, alkyle en C₁₋C₄, alcoxy en C₁₋C₄, trifluorométhyle, halogène
R¹ et R² = alkyle en C₁-C₄, cycloalkyle, hétéroalkyle
ou
R¹ et R² représentent ensemble un groupe alkylène ayant 2-6 atomes de carbone dans lequel un groupe -CH₂- peut être remplacé par l'oxygène, l'azote ou le soufre, et le nombre des groupes -CH₂- est 0 (1-aryl-imidazolin-2-one) ou 1 (1-aralkyl-imidazolin-2-one)
ainsi que de leurs sels pharmaceutiquement utilisables pour la fabrication d'un médicament pour le traitement des états d'angoisse et de tension.

2. Utilisation des composés de formule générale (I) selon la revendication 1 pour la fabrication d'un médicament pour le traitement des états d'angoisse dus à des conflits.
